# EUROPEAN PATENT SPECIFICATION

(11) **EP 3 012 634 B1**
(45) Date of publication and mention of the grant of the patent: **02.01.2019**
(21) Application number: 14814471.0
(22) Date of filing: 20.06.2014
(51) Int. Cl.: G01N 33/68, G01N 33/50, G01N 33/48, G01N 33/563

(54) **BIOMARKER FOR RHEUMATOID ARTHRITIS DIAGNOSIS OR ACTIVITY EVALUATION**
BIOMARKER ZUR DIAGNOSE RHEUMATOIDER ARTHRITIS ODER ZUR AKTIVITÄTSBEWERTUNG
BIOMARQUEUR DESTINÉ AU DIAGNOSTIQUE DE LA POLYARTHRITE RHUMATOÏDE OU À L'ÉVALUATION DE L'ACTIVITÉ

(30) Priority: 21.06.2013 KR 20130071739
(43) Date of publication of application: 27.04.2016
(73) Proprietor: Catholic University Industry-Academic Cooperation Foundation, Seocho-gu Seoul 137-701 (KR)
(72) Inventor: KIM, Wan-Uk, Seoul 137-852 (KR); HWANG, Daehee, Pohang-si Gyeongsangbuk-do 791-750 (KR); YI, Eugene C, Seoul 151-848 (KR); KANG, Min Jueng, Seoul 110-799 (KR); PARK, Yune Jung, Suwon-si Gyeonggi-do 442-703 (KR); YOU, Sung youg, Pohang-si Gyeongsangbuk-do 790-824 (KR)
(74) Representative: Schiweck Weinzierl Koch Patentanwälte Partnerschaft mbB
(86) International application number: PCT/KR2014/005488
(87) International publication number: WO 2014/204274

(56) References cited:
- WO-A2-2004/082617
- KR-A- 20120 104 517
- US-A1- 2007 148 704
- US-A1- 2010 297 682
- US-A1- 2012 083 423
- T. R. MIKULS ET AL: "Soluble CD14 and CD14 Polymorphisms in Rheumatoid Arthritis", JOURNAL OF RHEUMATOLOGY, vol. 38, no. 12, 1 December 2011 (2011-12-01), pages 2509-2516, XP055320804, CA ISSN: 0315-162X, DOI: 10.3899/jrheum.110378
- YU S ET AL: "Pathological significance of elevated soluble CD14 production in rheumatoid arthritis: In the presence of soluble CD14, lipopolysaccharides at low concentrations activate RA synovial fibroblasts", RHEUMATOLOGY INTERNATIONAL, vol. 17, no. 6, April 1998 (1998-04), pages 237-243, XP002764418, ISSN: 0172-8172
- BAS SYLVETTE ET AL: "CD14 is an acute-phase protein.", JOURNAL OF IMMUNOLOGY (BALTIMORE, MD. : 1950) 1 APR 2004, vol. 172, no. 7, 1 April 2004 (2004-04-01) , pages 4470-4479, XP002764419, ISSN: 0022-1767
- SMILJANOVIC BILJANA ET AL: "SYNOVIAL TISSUE TRANSCRIPTOME AND SYNOVIAL FLUID PROTEOME HAVE STRONGER DISCRIMINATORY POWER THAN SERUM IN DISSECTING INFLAMMATION IN RA", ANNALS OF THE RHEUMATIC DISEASES, vol. 72, no. Suppl. 1, March 2013 (2013-03), page A81, XP002764420, & 33RD EUROPEAN WORKSHOP FOR RHEUMATOLOGY RESEARCH (EWRR); PRAGUE, CZECH REPUBLIC; FEBRUARY 28 -MARCH 02, 2013
- PARK YUNE-JUNG ET AL: "Discovery Of Novel Biomarkers In Rheumatoid Urine: Significance Of Urinary CD14", ARTHRITIS & RHEUMATISM, vol. 65, no. Suppl. 10, Sp. Iss. SI, October 2013 (2013-10), page S1032, XP002764421, & 77TH ANNUAL MEETING OF THE AMERICAN-COLLEGE-OF-RHEUMATOLOGY / 48TH ANNUAL MEETING OF THE ASSOCIATION; SAN DIEGO, CA, USA; OCTOBER 25 -30, 2013
- AHMED FARID E: "The role of capillary electrophoresis-mass spectrometry to proteome analysis and biomarker discovery.", JOURNAL OF CHROMATOGRAPHY. B, ANALYTICAL TECHNOLOGIES IN THE BIOMEDICAL AND LIFE SCIENCES 15 JUL 2009, vol. 877, no. 22, 15 July 2009 (2009-07-15), pages 1963-1981, XP026224076, ISSN: 1873-376X
- WILLIAMS ADAM ET AL: "Applications of proteomics in cartilage biology and osteoarthritis research.", FRONTIERS IN BIOSCIENCE (LANDMARK EDITION) 2011, vol. 16, 2011, pages 2622-2644, XP002764422, ISSN: 1093-4715
- MIN JUENG KANG ET AL: "Urinary Proteome Profile Predictive of Disease Activity in Rheumatoid Arthritis", JOURNAL OF PROTEOME RESEARCH., vol. 13, no. 11, 7 November 2014 (2014-11-07), pages 5206-5217, XP055274896, US ISSN: 1535-3893, DOI: 10.1021/pr500467d
- VITOR HUGO TEIXEIRA ET AL: "Transcriptome Analysis Describing New Immunity and Defense Genes in Peripheral Blood Mononuclear Cells of Rheumatoid Arthritis Patients", PLOS ONE, vol. 4, no. 8, 27 August 2009 (2009-08-27) , page e6803, XP055300509, DOI: 10.1371/journal.pone.0006803
- CECILIANI FABRIZIO ET AL: "The acute phase protein alpha1-acid glycoprotein: a model for altered glycosylation during diseases.", CURRENT PROTEIN & PEPTIDE SCIENCE FEB 2007, vol. 8, no. 1, February 2007 (2007-02), pages 91-108, XP002768235, ISSN: 1389-2037
- TEIXEIRA ET AL.: 'Transcriptome analysis describing new immunity and defense genes in peripheral blood mononuclear cells of rheumatoid arthritis patients' PLOS ONE vol. 4, no. 8, 2009, pages 1 - 10, XP055300509
- DATABASE GENBANK [Online] 28 October 2004 KALNINE: 'OROSOMUCOID 1, PARTIAL', XP055300597 Retrieved from NCBI Database accession no. AV38575.1

## Description

### [Technical Field]

The present invention relates to a composition and kit for rheumatoid arthritis (RA) diagnosis or activity evaluation, the composition and kit each including an agent for measuring the concentration of at least soluble CD14 (sCD14), and a-1-acid glycoprotein 2 (AGP2). Further, the present invention relates to a use of the composition for diagnosing RA or evaluating activity.

### [Background Art]

RA is a disease that occurs due to the inflammation of the tissue called the synovium, which surrounds the joint, and is a typical chronic disease which is presumed to affect approximately 1% of the total population of Korea.

The diagnosis of RA primarily depends on clinical symptoms, but has a limitation in that the diagnosis is made after the joint is already quite damaged, and rheumatoid factor (RF) is included as a serological marker for RA in the diagnostic criteria by the American College of Rheumatology (ACR), which are the international diagnostic criteria, but RF has disadvantages in that RF shows that in that 20% of the patients with RA tested negative for RF throughout the progression of the disease, and thus, RF has a problem in sensitivity, and RF appears in patients with other rheumatic diseases or chronic inflammations and malignant tumors and even in some healthy seniors, and thus has low specificity.

Further, as a medical treatment method of RA, in which an analgesic anti-inflammatory drug is generally administered in combination with various anti-rheumatic agents, is used in order to minimize the damage to the joint, prevent loss of functions, and reduce the pain. Biological therapeutic agents have been recently developed and used as a combination treatment with an antirheumatic drug, and when the severity of the disease is high, an operative therapy is carried out. However, the aforementioned treatment method may cause a continuous deformation of the joint in spite of significantly excellent effects and has difficulty in appropriately treating the disease in some cases due to the drug side effects, and has a disadvantage in that a lot of costs are required due to an increase in drug costs resulting from the development costs of new drugs, and thus, there is an urgent need for developing methods which may appropriately diagnose and predict the onset, prognosis and severity of RA.

Further, it is based on the expectation that the progression of the disease will be delayed or prevented from the viewpoint of destruction of tissue, loss of cartilage, and erosion of a joint to treat RA, and since the progression of RA results from the activation of factors associated with the cause of the disease, it is important to analyze the relationship of the progression of the disease with the activity closely. As an example, it has been reported that there is convalescence when the activity of the disease is periodically monitored (YPM Goekoop-Ruiterman et al., Ann. Rheum. Dis. 2009 (Epublication Jan. 20, 2009)).

As a method of measuring the activity of RA in the clinical field in the related art, methods of measuring the counts of tender and swollen 28 joints such as shoulders, elbows, wrists, knees, and fingers, the number of joints exhibiting pressure pain and swelling, the erythrocyte sedimentation rate (ESR) or inflammation levels such as C-reactive protein (CRP) have been used. However, since it is difficult to correctly reflect the overall activity of the disease only by each of the indices, a disease activity score 28 (DAS28), by which the activity of the disease is synthetically judged by combining the aforementioned indices has been developed.

However, since the DAS28 is measured by an invasive method, the physical checkup required for deriving the DAS28 of a patient causes pain, and it takes a lot of time to perform the physical checkup. In addition, an experienced evaluator is needed to minimize a wide operator variability in order to accurately determine the DAS28 of a patient, and therefore, there is a problem in that it is limited to utilize the DAS28.

### [Disclosure]

### [Technical Problem]

Accordingly, a technology, which may appropriately diagnose and predict the onset, prognosis and severity of RA, need be developed. Further, in order to select an optimal therapy for treating a patient and judge whether the patient exhibits a treatment response appropriate for the corresponding treatment, an objective disease activity status marker which reflects the response for the treatment is needed.

Thus, the present inventors developed a biomarker for easily diagnosing RA and accurately and clinically evaluating the disease activity by using a urine sample, and used the biomarker to specifically quantify and evaluate the disease activity of a patient, and understand the therapeutic effects which affect the activity of the disease in order to maximize the therapeutic benefits of individual patients, thereby allowing better therapeutic effects to be exhibited.

### [Technical Solution]

An object of the present invention is to provide a kit for RA diagnosis or activity evaluation, including agents to measure the concentration of sCD14 and AGP2 in combination.

Furthermore, an object of the present invention is to provide a method for providing information for diagnosing RA or evaluating activity, the method including: measuring the concentrations of sCD14, and AGP2 from a urine sample.

Further, an object of the present invention is to provide a method for diagnosing RA or evaluating activity, the method including: measuring the concentrations of sCD14 and AGP2 in combination in a urine sample.

### [Advantageous Effects]

The present invention allows a treatment suitable for the state of a patient to be performed by using an agent for measuring the concentrations of sCD14, and AGP2 to diagnose RA or measure the activity of the disease.

### [Description of Drawings]

FIG. 1 illustrates that albumin is removed from each urine sample in order to improve detection of urinary proteins from patients with RA and osteoarthritis (OA), and then the urine sample is divided into 13 fractions by using an SDS-PAGE gel.
FIG. 2(A) illustrates a proteomic access method for discovering a protein which exhibits a difference in the expression from urine samples obtained from RA and OA patients, FIG. 2(B) illustrates that proteins separated from urine samples obtained from RA and OA patients are classified according to the function, and FIG. 2(C) illustrates that proteins separated from urine samples obtained from RA and OA patients are classified according to the origin.
FIG. 3 illustrates proteins which are particularly highly expressed in RA patients by using ELISA, and FIG. 3(A) illustrates the result for gelsolin (GSN), FIG. 3(B) illustrates the result for a-1-acid glycoprotein 1 (AGP1), FIG. 3(C) illustrates the result for a-1-acid glycoprotein 2 (AGP2), and FIG. 3(D) illustrates the result for sCD14.

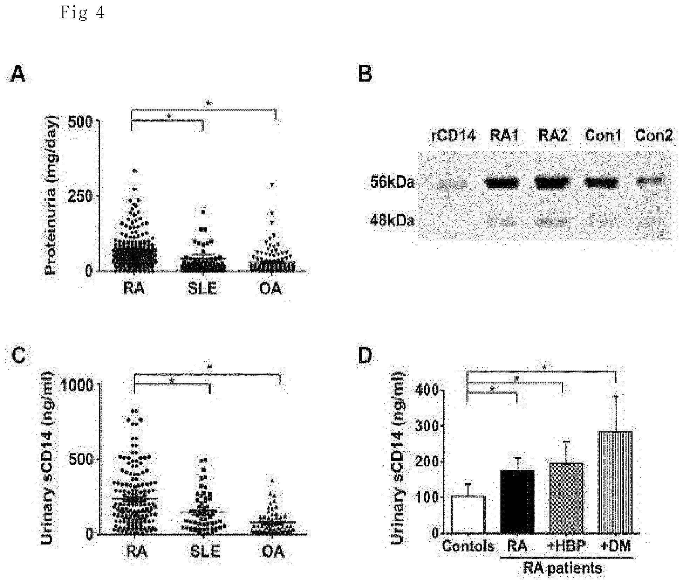

FIG. 4 is an observation of the concentration of sCD14 in samples obtained from patients with RA, OA, and systemic lupus erythematosus (SLE), and FIG. 4(A) illustrates comparison of the degrees of proteinuria among the groups of patients and FIG. 4(B) illustrates whether glycophosphatidylinositol (GPI)-free sCD14 (48 kDa) or GPI-linked sCD14 (56 kDa) is detected from the RA urine sample by using Western blotting. FIG. 4(C) illustrates comparison of the amounts of sCD14 in the urine among the groups of RA, OA, and SLE patients, and FIG. 4(D) illustrates the amounts of sCD14 in the urine in which diabetes mellitus (DM) and high blood pressure (HBP) are corrected from OA patients (control) and RA patients.
FIG. 5 illustrates the comparison of the concentrations of serum sCD14 between the RA and OA patients.
FIG. 6 illustrates the correlation of urinary sCD14 and serum sCD14 levels with clinical variables.
FIG. 7 illustrates the concentration of urinary sCD14 according to the disease activity status by DAS28.
FIG. 8 illustrates the correlation between urinary sCD14 and serum sCD14.
FIG. 9 illustrates the probability that urinary sCD14 is produced at high concentration according to the serum sCD14 level (upper panel) and the concentration of proteinuria (lower panel).
FIG. 10 illustrates the correlation between urine sCD14 and proteinuria.
FIG. 11 illustrates receiver operating characteristic (ROC) curves for testing high disease activity according to the concentrations of urinary sCD14, C-reactive protein (CRP), hemoglobin, platelet, and albumin.
FIG. 12 illustrates the correlation of sCD14, AGP1, AGP2, sCD14, and GSN protein with ESR, CRP, DAS28, hemoglobin, the number of white blood cells (WBCs), and albumin in the urine sample obtained from RA patients.
FIG. 13 illustrates the correlation of sCD14, AGP1, AGP2, sCD14, and GSN protein with ESR, CRP, DAS28, hemoglobin, the number of white blood cells (WBCs), and albumin in the urine sample obtained from control patients.
FIG. 14 illustrates ROC curves for testing high disease activity status according to the concentrations of urinary AGP2 and C-reactive protein (CRP).
FIG. 15 illustrates ROC curves for testing high disease activity status according to the concentrations of urinary sCD14, AGP1, AGP2, and GSN.
FIG. 16 illustrates ROC curves for testing high disease activity status according to various combinations of urinary sCD14, AGP1, and AGP2.

### [Best Mode]

As one aspect to achieve the object, the present invention relates to a composition for RA diagnosis or activity evaluation, including an agent for measuring the concentrations of one or more proteins selected from the group consisting of soluble CD14 (sCD14), AGP1, and AGP2.

In the present invention, the term "diagnosis" means confirming the presence or characteristic of the pathological state. For the purpose of the present invention, the diagnosis means confirming whether RA occurs, or furthermore, may mean confirming whether the disease proceeds or becomes aggravated.

In the present invention, the term "a marker for diagnosis, a marker for diagnosing, or a diagnosis marker" is a material which may diagnose RA by distinguishing RA from the states other than RA (for example, distinguishing RA from the normal state, or distinguishing RA from other arthritis such as OA), and includes organic biomolecules such as polypeptides or nucleic acids (for example, mRNA and the like), lipids, glycolipids, glycoproteins or sugars (monosaccharide, disaccharide, oligosaccharide, and the like), which show an increase or decrease in a sample obtained from individuals with RA compared to the samples obtained from individuals who do not suffer from RA. For the purpose of the present invention, the diagnosis marker of the present invention refers to AGP1 and AGP2, which exhibit expression specifically increased in samples obtained from patients with RA, and to sCD14 protein which exhibits expression specifically decreased in samples obtained from patients with RA.

More specifically, the present invention provides sCD14, and AGP2 as a diagnosis marker of RA.

In the present invention, "the activity of RA" or "the disease activity status of RA" means the overall degree of inflammation of a patient with RA, or the progression degree of RA, and may be usefully used in evaluating the response to treatment or the presence or absence of remission.

Further, in the present invention, "the evaluation of the activity of RA" refers to evaluation of the overall degree of inflammation of a patient with RA, or the progression degree of RA, and furthermore, is a concept which includes judging whether a treatment currently performed is effective for the patient based on the evaluation, selecting an optimal therapy (pharmacotherapy or operation, and the like), and predicting responsiveness to the treatment and whether the disease is alleviated.

The DAS28, which is a representative method of measuring the activity of RA in the clinical field in the related art, or the DAS28, which is a modified form thereof, is an index evaluation method by a point calculation system, and evaluates subjective factors which patients and doctors evaluate along with some objective factors such as an inflammation index test or radiological finding. The DAS28 is a composite index composed of the number of joints feeling pressure pain and the number of joints exhibiting edema among 28 joints of a patient, the erythrocyte sedimentation rate, and a systemic evaluation of the patient, and the 28 joints include joints of both shoulders, elbow and wrist joints, metacarpophalangeal joints, proximal interphalangeal joints, knee joints, and the like.

The disease activity status of RA examined by the DAS28 may be calculated at 0 point up to 9.4 point, and in general, in terms of the DAS28 score, a point of less than 2.6 is defined as little disease activity (remission), a point of 2.6 or more and less than 3.2 is defined as a low disease activity (mild case), a point of 3.2 or more and less than 5.1 is defined as a moderate disease activity (moderate case), and a point of 5.1 or more is defined as a high disease activity (severe case). However, as mentioned above in the Background Art, the disease activity status evaluation method in the related art by DAS28 is invasive, and thus has a problem in that pain is incurred by a patient and it takes a lot of time.

The present invention is characterized to provide an objective disease activity status marker which may non-invasively confirm a current disease activity status of a patient without resorting to a RA activity evaluation such as DAS28 in the related art, and may select an optimal therapy for the patient based on the current disease activity status and judge whether a treatment response appropriate for the corresponding treatment is exhibited.

More specifically, the present invention provides sCD14 and AGP2 as a disease activity status marker of RA. In the present invention, the proteins are indices which reflect the disease state of RA and exhibit a higher concentration in an active group of RA than in a non-active group of RA, and exhibit not only a strong positive correlation with the disease activity status evaluation means in the related art, such as DAS28 currently used for disease activity status evaluation in the clinical field, but also a significant correlation with CRP used as an important inflammation activity marker when the DAS28 is evaluated.

Accordingly, sCD14, AGP1 and/or AGP2 are(is) a non-invasive biomarker, and may be usefully used for evaluating an inflammation activity response from a patient in itself and forecasting a treatment response. In addition, sCD14, AGP1 and/or AGP2 may also be used as an auxiliary marker for judging the treatment response when an inflammation activity marker such as CRP and ESR exhibits a result different from the clinical aspect of a patient with RA. In particular, it is expected that when used in combination with a serum CRP evaluation, sCD14, AGP1 and/or AGP2 may be used as a more useful biomarker.

In the present invention, "sCD14" is a soluble CD 14 which is one of the forms of CD14. CD14 is a glycophosphatidylinositol (GPI)-linked membrane surface protein of 55 kDa, and for the amino acid sequence of sCD14, the sequence information thereof may be confirmed from the gene database publicly known. For example, the amino acid sequence of human sCD14 protein may be confirmed from the NCBI Genbank Accession No. NP_000582.1, and is described in SEQ ID. 1.

In the present invention, "a-1-acid glycoprotein 1 (AGP1)" and "a-1-acid glycoprotein 2 (AGP2)" are also referred to as orosomucoids 1 and 2, respectively, and are proteins which are derived in a stress state such as infection, and secreted into the blood plasma under a stress condition such as infection and inflammation. In the present invention, it was found out that the protein is associated with activity of RA. For the amino acid sequences of AGP1 and AGP2, the sequence information thereof may be confirmed from the gene database publicly known. For example, the amino acid sequence of human AGP1 protein may be confirmed from the NCBI Genbank Accession No. NP_000598.2, and the amino acid sequence of human AGP2 protein may be confirmed from the NCBI Genbank Accession No. NP_000599.1. The amino acid sequence of AGP1 protein is described in SEQ ID. 2, and the amino acid sequence of AGP2 protein is described in SEQ ID. 3.

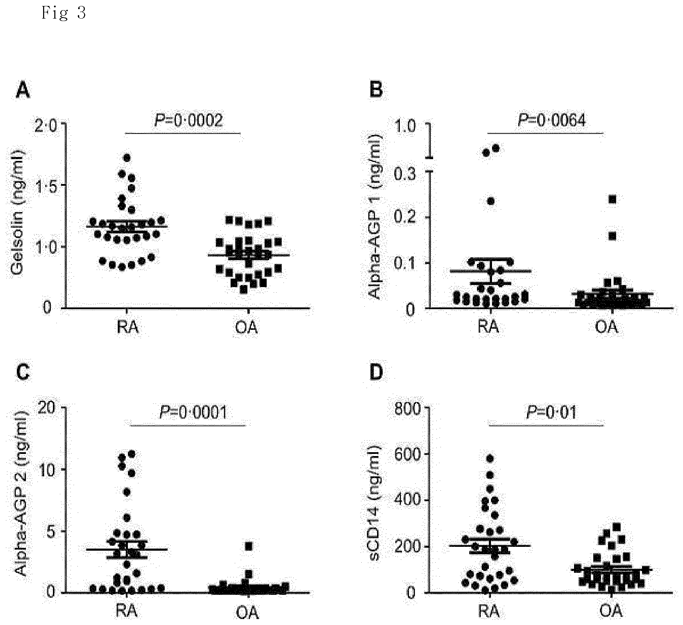

In a specific exemplary embodiment of the present invention, 12 proteins specifically exhibiting a difference in the expression from urine samples obtained from patients with RA and osteoarthritis (OA) were sorted out (IGHM, GSN, AGP1, SERPINA3, AGP2, CD14, AZGP1, COTL1, HPR, SERPINA7, CTSA, and GNS), and it was confirmed that among them, the amounts of particularly sCD14, AGP1, and AGP2 proteins were notably higher in RA patients than in OA patients (FIG. 3). Since these proteins have a certain change in the proteome profile, it was proven that these proteins are urinary protein biomarkers for RA diagnosis.

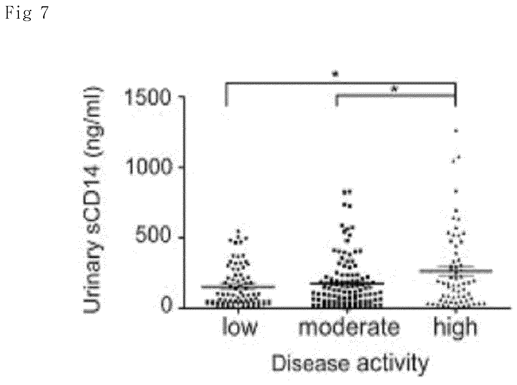

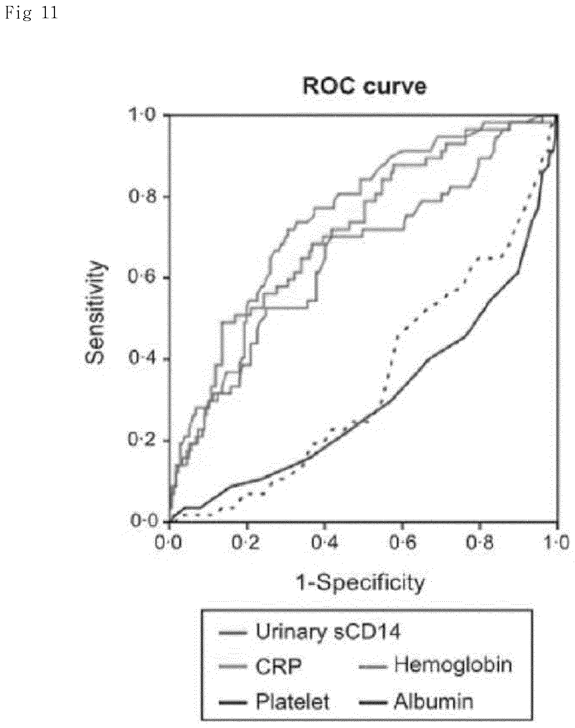

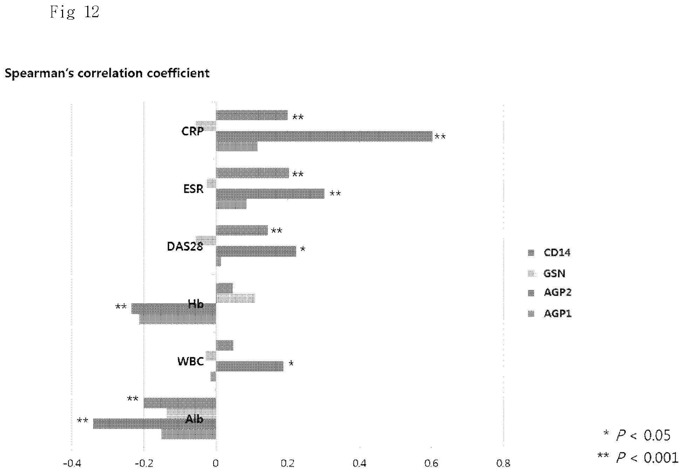

Further, in a specific exemplary embodiment of the present invention, when the RA patients were classified into three groups of high/moderate/low by the disease activity status according to the DAS28, the concentration of urinary sCD14 was increased in proportion to the DAS28 (FIG. 7), and thus, it could be seen that the urinary sCD14 level may be used as a sensitive measure for the activity of inflammatory response. In addition, as a result of carrying out an ROC analysis for diagnostic power of urinary sCD14 from RA patients having high disease activity (DAS28 > 5.1), the urinary sCD14 was at a level similar to that of CRP when differentiating high disease activity (DAS28 > 5.1), and the urinary sCD14 exhibited higher sensitivity in a region exhibiting a specificity of 80% or more than CRP (FIG. 11), and thus, it could be confirmed that the disease activity status could be predicted more complementarily than the case when the urinary sCD14 was measured alone or in combination with CRP. It was confirmed that the urinary sCD14 has a sensitivity and a specificity of 68.4% and 62.7%, respectively, which predict high disease activity status of RA patients (when the cut-off was set to 0.06), and thus, the urinary sCD14 was proven to be feasible as a biomarker. AGP1 and AGP2 also exhibited correlation with CRP (FIG. 12), and it was confirmed that particularly urinary AGP2 has a sensitivity and a specificity of 71.6% and 70.0%, respectively, which forecast high disease activity status of RA patients (when the cut-off was set to 0.3555), and thus, the urinary AGP2 was proven to be feasible as a biomarker.

Furthermore, in the evaluation of RA disease activity status by (i) a combination of sCD14, AGP1, and AGP2, (ii) a combination of sCD14 and AGP2, or (iii) a combination of AGP1 and AGP2, both the sensitivity and the specificity were exhibited to be high, and thus, the proteins were proven to be feasible as biomarkers by the mutual combinations of the respective proteins.

Accordingly, it was confirmed that AGP1, AGP2, and sCD14 may be used as non-invasive biomarkers for diagnosing RA or evaluating disease activity status, and the mutual combination of these proteins are much better used as biomarkers.

As a preferred exemplary embodiment, the composition of the present invention may be a composition for RA diagnosis, further including an agent for measuring the concentration of one or more proteins selected from a group consisting of immunoglobulin heavy constant mu (IGHM; Uniprot. Accession No.: P01871), gelsolin (GSN; NCBI Genbank Accession No.: NP_000168.1), serpin peptidase inhibitor, clade A (alpha-1 antiproteinase, antitrypsin), member 3 (SERPINA3; NCBI Genbank Accession No.: NP_001076.1), alpha-2-glycoprotein 1, zinc (AZGP1; NCBI Genbank Accession No.: NP_001176), coactosin-like F-actin binding protein 1 (COTL1; NCBI Genbank Accession No.: NP_066972), haptoglobin isoform 2 preproprotein (HPR; NCBI Genbank Accession No.: NP_001119574), serpin peptidase inhibitor, clade A (alpha-1 antiproteinase, antitrypsin), member 7 (SERPINA7; NCBI Genbank Accession No.: NP_000345), cathepsin A (CTSA; NCBI Genbank Accession No.: NP_000299), and glucosamine(N-acetyl)-6-sulfatase (GNS; NCBI Genbank Accession No.: NP_002067).

As still another preferred aspect, the agent for measuring the concentrations of the proteins may be an antibody specific to sCD14, AGP1, and AGP2.

In the present invention, "the antibody" means a protein molecule specific to an antigenic site. For the purpose of the present invention, the antibody means an antibody specifically bound to the marker protein sCD14, AGP1, and/or AGP2, and includes a monoclonal antibody, a polyclonal antibody, and a recombinant antibody.

A monoclonal antibody may be prepared by using a hybridoma method (Kohler and Milstein(1976) European Journal of Immunology 6:511-519) or a phage antibody library (Clackson et al., Nature, 352: 624-628, 1991; Marks et al., J. Mol. Biol., 222: 58, 1-597, 1991) technique, which is publicly known in the art.

A polyclonal antibody may be produced by a method publicly known in the art, which includes injecting the aforementioned protein antigen into an animal, and collecting blood from the animal to obtain serum including antibodies. Such a polyclonal antibody may be prepared from any animal species host, such as goats, rabbits, sheep, monkeys, horses, pigs, cows and dogs.

In addition, the antibody of the present invention also includes a special antibody such as a chimeric antibody, a humanized antibody and a human antibody.

Furthermore, the antibody used in the present invention includes not only a complete form having two full-length light chains and two full-length heavy chains, but also the functional fragments of the antibody molecule. The functional fragments of the antibody molecule refer to the fragments having at least a function of binding antigens, and examples thereof include Fab, F(ab'), F(ab') 2, Fv, and the like.

A method for measuring the concentrations of sCD14, AGP1 and/or AGP2 in a sample by using these antibodies may be used without limitation as long as the method is a method which may confirm the degree of producing an antigen-antibody complex by treating the sample with the antibody. The "antigen-antibody complex" means a binding product of sCD14, AGP1 and AGP2 proteins to an antibody specific thereto, and the quantity of antigen-antibody complex formed may be quantitatively measured through the signal size of a detection label.

For example, Western blot, enzyme linked immunosorbent assay (ELISA), immunoprecipitation assay, complement fixation assay, fluorescence activated cell sorter (FACS), or protein chip, and the like may be exemplified, but the present invention is not limited thereto.

As another aspect, the present invention relates to a kit for RA diagnosis or activity evaluation, including the composition for RA diagnosis or activity evaluation.

The kit of the present invention may include not only an agent which may measure the concentrations of sCD14, AGP1 and/or AGP2 in a patient sample, but also one or more compositions, solutions or devices suitable for concentration analysis. For example, the kit may include a substrate, a suitable buffer solution, a secondary antibody labeled with a detection label, a chromogenic substrate, and the like for an immunological detection of the antibody.

As a specific example, the kit for RA diagnosis or activity evaluation may be a kit which is characterized to include an essential element required for performing an ELISA, in order to implement various ELISA methods such as an ELISA kit and a sandwich ELISA. The ELISA kit includes an antibody specific to the proteins. The antibody is an antibody which has high specificity and affinity for sCD14, AGP1 and AGP2 proteins and minimal cross-reactivity to other proteins, and may be a monoclonal antibody, a polyclonal antibody, or a recombinant antibody. Further, the ELISA kit may include an antibody specific to a control protein. The other ELISA kits may include a reagent which may detect a bound antibody, for example, a labeled secondary antibody, a chromopore, an enzyme and a substrate thereof, or other materials which may be bound to the antibody, and the like.

In addition, the kit may be a kit for implementing Western blot, immunoprecipitation assay, complement fixation assay, fluorescence activated cell sorter, or protein chip, and the like, and may additionally include an additional configuration suitable for each analysis method. Through the analysis methods, it is possible to diagnose RA or evaluate activity by comparing the amount of antigen-antibody complex formed, and correspondingly, a patient may be appropriately treated.

Also disclosed is a protein chip for RA diagnosis or activity evaluation, including the composition for RA diagnosis or activity evaluation.

The protein chip is immobilized at high density because one or more antibodies to sCD14, AGP1 and AGP2 proteins are arranged at a predetermined position. The method for analyzing samples by using a protein chip may diagnose RA or confirm the activity by isolating a protein from a subject sample, hybridizing the separated protein with a protein chip to form an antigen-antibody complex, and reading this to confirm the degree of expression of the protein.

The amount of antigen-antibody complex formed may be quantitatively measured through the signal size of a detection label.

The detection label may be selected from a group consisting of an enzyme, a fluorescent material, a ligand, a luminescent material, a microparticle, a redox molecule, and a radioisotope, but is not necessarily limited thereto.

As still another aspect, the present invention relates to a method for providing information for diagnosing RA or evaluating activity, the method including: measuring the concentrations of sCD14, and AGP2 from a urine sample.

As still yet another aspect, the present invention relates to a use of measuring the concentrations of sCD14, and AGP2 in combination in a urine sample for diagnosing RA or evaluating activity.

In the present invention, the term "subject sample" includes a sample such as a tissue, a cell, whole blood, blood plasma, serum, blood, saliva, sputum, lymph, cerebrospinal fluid, interstitial fluid, or urine, which exhibits a difference in the expression levels of sCD14, AGP1, and AGP2, which are marker proteins, but is not limited thereto.

The subject sample is a urine sample.

The procedure of isolating the protein from a subject sample may be performed by using a process publicly known, and the protein level may be measured by the aforementioned various methods for measuring an antigen-antibody complex.

As a more preferred aspect, in the present invention, the concentration of the protein may be measured by using antibodies specific to sCD14, AGP1, and AGP2.

As a much more preferred aspect, the antibody may be a monoclonal antibody or a polyclonal antibody, which is specific to sCD14, AGP1, and AGP2, but furthermore, may be a functional fragment of an antibody having antigen binding activity.

In the present invention, the concentrations of sCD14, AGP1, and/or AGP2 which are(is) a marker protein of rheumatoid arthritis may be measured by using an antibody to diagnose rheumatoid arthritis, so that it is possible to select an optimal therapy so as to be suitable for the status of a patient by evaluating the activity of the disease, and it is possible to judge whether the patient exhibits an appropriate treatment response to the corresponding treatment.

Hereinafter, the present invention will be described in detail through the Examples. However, the following Examples are only for exemplifying the present invention, and the present invention is not limited by the following Examples.

### Example 1. Experimental Materials and Experimental Methods

### 1-1. Selection of Subject Patient

Patients diagnosed with rheumatoid arthritis (RA) were selected in accordance with the American College of Rheumatology (ACR) criteria in 1987. In the patient medical records, age, gender, height, body weight, duration of disease and the like were respectively confirmed. Clinical factors, such as complete blood count (CBC), blood sugar, serum creatinine, serum albumin, erythrocyte sedimentation rate (ESR), C-reactive protein (CRP), rheumatoid factor (RF), and anti-cyclic citrullinated protein (CCP) antibodies which are antibodies specific for the diagnosis of RA, were evaluated. However, current smokers and patients with a history of a cardiovascular disease, hypertension patients who are not controlled (> 160/100 mmHg), patients with rheumatoid vasculitis, amyloidosis, diabetic nephropathy, or chronic kidney disease (glomerular filtration rate < 60 ml/min/1.732), patients with overt or chronic infection, patients with thyroid gland or liver disease, cancer, and patients who are pregnant were excluded from the present study. A stable treatment for 3 months was also needed for the present study. Patients, who had not taken angiotensin-converting enzyme inhibitors or angiotensin receptor blockers among hypertension drugs, were included. Both hands and both feet of the RA patients were X-ray photographed, and these data were subjected to analysis while the medical specialists were ignorant of the status or conditions of each of the patients. The bone erosion was measured and the graphical severity was analyzed by the Sharp/van der Heijde method. The study protocol was approved by the Institutional Review Board of the Catholic Medical Center (XC09TIMI0070), and an experiment after all the written consents for the study protocol were received from all the patients, was carried out.

274 RA patients, and 120 patients with osteoarthritis (OA) and 60 patients with systemic lupus erythematosus (SLE) as a control were recruited. The characteristics of the subject patients are shown in the following Table 1.

**[Table 1]**

| **Variables** | **RA** (n = 274) | **OA** (n = 120) | **SLE** (n=60) |
|---|---|---|---|
| Age (year) | 53.2±2.4 | 52.9±0.2 | 43.9±3.2 |
| Women (%) | 224(81.8) | 98(81.8) | 55(91.7) |
| Hypertension (%) | 71(25.9) | 34(28.3) | 16(26.7) |
| Diabetes mellitus (%) | 29(10.6) | 14(11.7) | 4(6.7) |
| Creatinine (mg/dl) | 0.7±0.2 | 0.8±0.1 | 0.8±0.4 |
| Glomerular filtration rate | 99.5 [85.8-114.7] | 97.5 [81.1-115.3] | 93.2 [80.1-97.8] |
| Erythrocyte sedimentation rate (mm/hr) | 39.6±7.7 | 15.7±0.9 | 28.6±0.4 |
| C-reactive protein (mg/l) | 12.8 [1.2-36.7] | 0.8 [0.5-1.4] | 0.9 [0.5-3.2] |
| Number of white blood cells (/ml) | 7772.6±279.7 | 6046.9±713.8 | 5171.6±246.3 |
| Hemoglobin (mg/dl) | 12.6±1.3 | 13.1±1.1 | 11.6±1.6 |
| Number of platelets (/ml) | 285.1±90.9 | 244.7±53.2 | 195.1±81.5 |
| Albumin (mg/dl) | 4.2±0.4 | 4.6±0.2 | 3.9±0.5 |
| Globulin (mg/dl) | 3.2±0.5 | 2.8±0.4 | 3.4±0.1 |
| Duration of disease (year) | 5.0 [2.0-12.0] | 2.0 [0.0-4.0] | 4.0 [2.0-7.3] |
| Non-steroidal anti-inflammatory drug (%) | 169(61.7) | 81(67.5) | 29(48.3) |
| Prednisolone (%) | 201(73.4) | 13(10.8) | 21(35.0) |

### 1-2. Extraction of Protein from Urine Sample

A urine sample was collected during the routine examination of a patient. Albumin and creatine discharged from the urine were measured by using Hitachi 7600-110 colorimetry. The collected urine sample was stored at -80°C. Urine samples were obtained from 20 patients with RA (total 100 ml) and 19 patients with OA (total 95 ml), and centrifuged under the conditions of 2,000 g and 4°C for 5 minutes in order to remove the unnecessary debris. The samples from the patients with OA were used as a normal control for RA. The urine sample was mixed with methanol at a ratio of 1:9 (v/v), incubated at -20°C for 14 hours, and then centrifuged under the conditions of 14,000 g and 4°C for 30 minutes, thereby extracting proteins. A protein pellet was washed with methanol, dried in the air, and then re-suspended in a tris buffer solution (5 mM EDTA, 50 mM Tris-HCl pH 7.5). The amount of the protein was measured by using a Micro BCA Protein Assay Kit (Thermo Scientific, Rockford, IL, USA).

### 1-3. Removal of Albumin and Dialysis

In order to improve the detection of urinary protein, albumin was removed by using Affinity Removal Spin Cartridge, Human Albumin (Agilent Technologies, Wilmington, DE, USA). According to the manufacturer's protocol, a urinary protein sample was three-fold diluted with depletion buffer A (Agilent Technologies), and then filtered with a 0.22 µm spin filter (Agilent Technologies). The diluted urinary sample was loaded into the spin cartridge, and then centrifuged under 100 g for 1.5 minutes. The flow-through fraction was collected, and centrifuged along with a depletion buffer A under 100 g for 2.5 minutes to wash the cartridge. All the flow-through fractions were mixed. The urine sample from which albumin had been removed was dialyzed by using Slide-A-Lyzer Dialysis Cassettes (Thermo Scientific, a molecular weight cut-off of 3.5 kDa) to remove the salts. In order to reduce the volume of the dialyzed sample, the sample was speed-vac dried. The amount of the protein was measured by using a Micro BCA Protein Assay Kit (Thermo Scientific, Rockford, IL, USA).

### 4. SDS-PAGE and in-gel digestion

The protein sample from which albumin had been removed was warmed in an LDS sample buffer (Invitrogen, Carlsbad, CA), fractionated in a 4 to 12% Bris-Tris NuPAGE gel (Invitrogen), and stained with Coomassie Brilliant Blue (Sigma-Aldrich). The entire gel lanes were cut into 13 pieces, and in-gel tryptic digestion was performed according to the general protocol. When briefly described, the cut protein band was discolored with acetonitrile (ACN) and 100 mM of ammonium bicarbonate (NH₄HCO₃) for 15 minutes. The protein in the gel was reduced with 20 mM of dithiothreitol, and alkylated with 55 mM of iodoacetamide and 100 mM of NH₄HCO₃. ACN was dehydrated, and then the protein was digested at 37°C overnight with 13 ng/µL sequencing grade modified procine trypsin (Promega, Madison, WI, USA) in 50 mM of NH₄HCO₃. Peptides were extracted from the gel pieces in 50% v/v ACN in 0.1% formic acid, vacuum-dried, and then stored at -20°C.

### 1-5. Mass Spectrometry Analysis

The dried peptide sample was dissolved in 100 µL of 0.1% formic acid, 2% ACN in water. The peptide sample extracted by in-gel digestion was subjected to LC-MS/MS analysis by using LTQ-velos (ThermoFinnigan, San Jose, CA), coupled on-line by nano-HPLC system (Easy nLC, Thermo Fisher Scientific), and then mounted to a reversed-phase microcapillary electrospray ionization system. A 5 µL peptide mixture was loaded onto an HPLC combined with an in-house packed Magic™ C₁₈ column (10 cm in length, 75 µm in inner diameter). Peptides were eluted at a flow rate of 300 nL/min from the HPLC column by sequentially using Buffer B (ACN : water : formic acid, 97.9 : 2 : 0.1 [v/v/v]) in Buffer A (water:ACN:formic acid, 97.9 : 2 : 0.1 [v/v/v]) with a concentration of 2 to 38%. The eluted peptides were directly sprayed from the top of the capillary column by LTQ-velos to be subjected to a mass analyzer analysis. The spray voltage was set to 1.9 kV, the temperature of the capillary tube was set to 200°C, and the normalized collision energy was adjusted to 35%. The LTQ was operated in a data-dependent mode, and a machine measured intensity of all peptide ions in a mass range of 400 to 1,400 (mass-to-charge ratios). The top five most intense ions were isolated for collision-induced dissociation.

### 1-6. Urine Proteome Data Analysis

MS/MS spectra were searched against the IPI human protein (version 3.86), and decoy databases was sequence-reversed by using SEQUEST of Bio-Works Browser rev 3.1. The SEQUEST search results were additionally analyzed by using PeptideProphet and ProteinProphet programs in the Trans Proteomic Pipeline (TPP) v 4.5. Peptides with FDR = 0.01 or a PeptideProphet probability = 0.9 were selected. Next, proteins with ProteinProphet probability ≥ 0.95 were identified. Among these proteins, the proteins, which were detected two or more times in the 52 profile data in RA or OA samples or had two or more sibling peptides, were selected as the high confidence proteins.

The urinary proteins were relatively quantified by using the APEX Quantitative Proteomics Tool. In each urine sample, a training data set of the 50 most abundant proteins under the conditions of protein probabilities≥ 0.95 and normalization factor = 1 was generated, then the predicted peptide count (Oi) of the urinary proteins was computed from the training set, and finally, the APEX abundances of the urinary proteins were calculated by using the protXML file and the urinary protein Oi values. In the urine samples obtained from the RA and OA patients, the relative APEX abundances were normalized by using the quantile normalization method. Proteins exhibiting a difference in expression were isolated by using the normalized APEX abundances. For each protein, the t-test and log₂-median-ratio test were applied to the respective abundances, and the procedure was repeated four times. The empirical null distributions were estimated in the two tests by substituting each result. P values corrected in the two tests were calculated by using the distribution for each protein, the P values were combined from the two tests, and the combined P value was calculated by using the Stouffer's method. Finally, the FDR for the combined p value was calculated by using the Storey's method. Proteins satisfying both FDR < 0.05 and fold-change ≥ 1.5 were selected as differentially expressed proteins (DEPs).

In order to explore cellular processes or intracellular localization of the high-confidence urinary proteins, GO biological processes and cellular components with P values of 0.05 or less, which were computed by DAVID software, were identified. When the cellular processes of 134 DEPs were identified, the functional annotation clustering was used in the KEGG pathway and GO biological process of DAVID software.

### 1-7. Transcriptome Data Analysis and Collection of Human Serum Proteomes

Gene expression data collected from synovial tissues (GSE12021, GSE1919, and GSE7307) and gene expression data collected from peripheral blood mononuclear cells (PBMCs) (GSE11827 and GSE15573) were obtained from the Gene Expression Omnibus. The intensities of the analysis were normalized by using the quantile normalization method. DEGs between OA and RA samples were determined by using the normalized intensities. The DEGs selected genes with FDRs < 0.05 and fold-changes ≥ 1.5. A high-confidence (FDR ≤ 0.01) list of 1929 proteins identified from 91 experiments including the human plasma proteome project (HPPP) phase I and II data was downloaded from the HPPP Data Central at PeptideAtlas.

### 1-8. Enzyme-Linked Immunosorbent Assay (ELISA)

For the quantification of sCD14 levels in serum and urine, ELISA was performed by using the CD14 ELISA assay (R&D Systems, Minneapolis, MN, USA). Even when the concentrations of GSN, AGP1, and AGP2 in urine were measured, the ELISA (USCN Life Science Inc., Missouri City, TX, USA) was used.

### 1-9. Statistical Analysis

The distribution for all variables was examined. Variables exhibiting a normal distribution were denoted as an average ± a standard deviation (SD), and variables exhibiting a non-normal distribution were denoted as a median value [interquartile range; IQR]. Comparisons among the three groups of patients were performed by a one-way analysis of variance (ANOVA) with Bonferroni correction as a post hoc test. For categorical data, the difference in prevalence was evaluated by a chi-square or Fisher's exact test. In the univariate analysis, Spearman correlation coefficients were used to display the correlation between urinary sCD14 and variables of interest. For the cross-sectional comparison between two groups, the Mann-Whitney U test was used. Cumulative probability plots were used in order to display high urinary sCD14 levels across patients with different baseline levels of serum sCD14 and proteinuria. For the forecast of disease activity status severity (high disease activity), ROC curves for urinary sCD14, CRP, hemoglobin, platelet and albumin levels were compared.

High urinary sCD14 means the top tertile of urinary sCD14. The ROC analysis was used in order to distinguish the high and low levels by determining optimal cut-off levels for ESR, CRP, GFR, creatinine, urinary sCD14, and the ratio of serum CRP to urinary sCD14. High ESR, high CRP, and impaired subclinical renal function are defined as baselines when the serum ESR level was ≥28 mm/hr, the serum CRP level was ≥ 0.5 mg/dL, GFR < 90 ml/min/1.73², and Cr ≥ 0.7 mg/dl.

All reported P values are two-tailed, with a P value of 0.05, indicating statistical significance. When the P values on both sides are less than 0.05, the values were considered as significant. The analysis was performed by using R software, version 2.14.1.

### Example 2. Urine Sample Protein Analysis

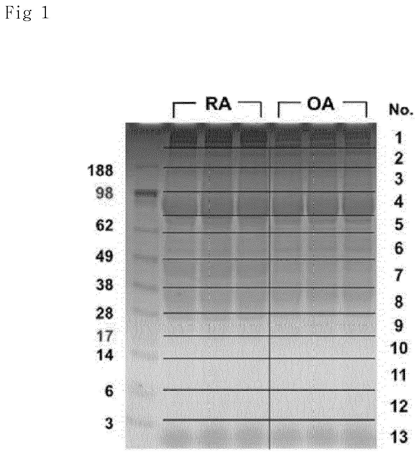

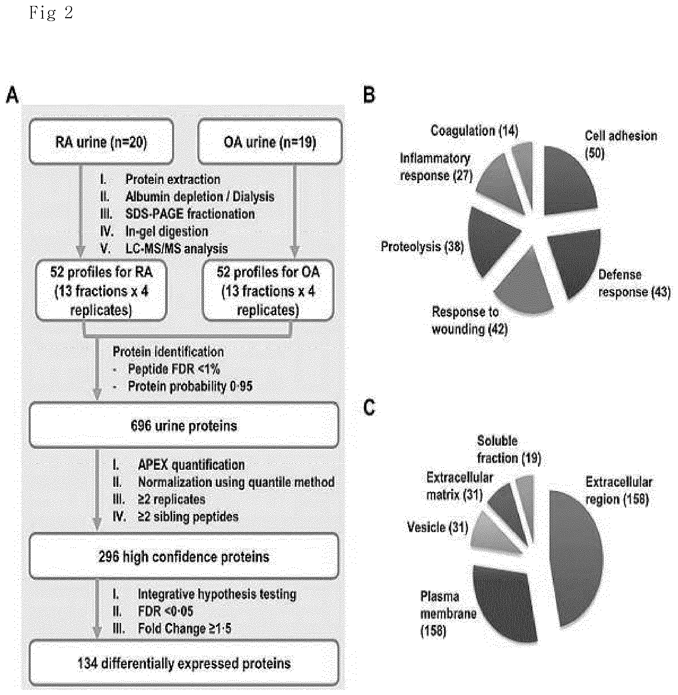

A urine sample was collected during the routine examination of a patient, and in order to improve the detection of urinary protein, albumin was removed by using affinity removal spin cartridge, human albumin (Agilent Technologies, Wilmington, DE, USA), and then the urine sample was separated into 13 fractions by using the SDS-PAGE gel (FIG. 1). And then, a general in-gel tryptic digestion was followed, and an LC-MS/MS analysis was performed for each fraction to prepare a total of 52 proteome profiles for each of the RA and OA samples. A protein analysis was performed by using the resulting MS/MS spectra, and IPI human version 3.86 and sequence-reversed decoy databases of SEQUEST were used. A total of 696 urinary proteins (586 and 556 proteins in OA and RA samples, respectively) were confirmed under the conditions of 0.01 of FDR peptide identification and 0.95 of Protein Prophet probability. The amounts of the proteins in RA and OA were determined by using the absolute protein expression (APEX) method (see methods for details). In order to determine a reliable urinary protein biomarker candidate group, 296 proteins, which were detected two or more times in each of the 52 profiles in RA or OA samples or had two or more sibling peptides, were observed in focus (FIG. 2(A)). In order to deeply understand the function and distribution levels of the urinary proteins found, 296 proteins, which represented intracellular actions and were reliable, were first examined by using DAVID software. These proteins are associated with cell adhesion, immune response (wound, defense, inflammation, and coagulation responses), and proteolysis (FIG. 2(B)). It was then examined that a protein representing intracellular elements originated from the extracellular region or matrix, and membrane associated elements. Through this, it was confirmed that urinary proteins were well separated from the urine sample (FIG. 2(C)).

It was confirmed that among the 296 high confidence proteins, 134 proteins were specifically expressed in the urine samples of RA patients compared to those of OA patients by using an integrative statistical method (DEPs; FDR < 0.05 and fold change of APEX abundance > 1.5), and among them, 12 proteins exhibiting a particularly significant difference in expression were selected (Table 2).

**[Table 2]**

| Protein | IPI Human database | P value | Difference in expression (RA/OA) |
|---|---|---|---|
| IGHM | IPI00896380.1 | 0.008925 | 1.076735 |
| GSN | IPI00026314.1 | 0.005315 | 1.267885 |
| AGP1 | IPI00022429.3 | 0.001132 | 1.907385 |
| SERPINA3 | IPI00550991.3 | 0.002952 | 1.794114 |
| AGP2 | IPI00020091.1 | 0.002113 | 1.636583 |
| CD14 | IPI00029260.2 | 0.030701 | 0.792891 |
| AZGP1 | IPI00166729.4 | 0.031514 | 0.596275 |
| COTL1 | IPI00017704.3 | 0.003831 | 1.417044 |
| HPR | IPI00478493.3 | 0.012258 | 0.96827 |
| SERPINA7 | IPI00292946.1 | 0.029417 | 0.950555 |
| CTSA | IPI00021794.8 | 0.039159 | 0.788813 |
| GNS | IPI00012102.1 | 0.007266 | 1.475078 |

As a result of confirmation by using the ELISA, among the 12 selected biomarker proteins, the amounts of GSN, AGP1, AGP2, and sCD14 proteins were notably higher in RA patients than those in OA patients (P < 0.01, FIG. 3). Since these proteins have a certain change in the proteome profile, it was proven that these proteins are urinary protein biomarkers for RA diagnosis.

And then, in order to illuminate sCD14 more profoundly, the experiment was performed by using 274 RA patients with, which was a larger group, and a control of 180 patients including 120 OA patients and 60 patients with systemic lupus erythematosus (SLE).

### Example 3. Measurement of Concentration of sCD14 from RA, OA, and SLE Patients

In RA patients, subclinical renal damage was relatively frequent, and microproteinuria and renal tubular disorder occurred. Accordingly, renal function and proteinuria degrees were first tested before the urinary sCD14 was measured from the group of RA patients. Proteinuria levels after correction for urine creatinine were significantly higher in RA patients (n = 274) than in OA patients (n = 120) or SLE patients (n = 60) with no renal disease (FIG. 4(A)). This means that subclinical renal damage (proteinuria <150 mg/day) frequently occurred in RA patients.

Further, proteinuria levels did not exhibit a large difference depending on whether prednisolone, nonsteroidal anti-inflammatory drugs (NSAIDs), disease-modifying anti-rheumatic drugs (DMARDs), and TNF inhibitors had been prescribed (Table 3).

The CD14 is a glycophosphatidylinositol(GPI)-linked membrane surface protein (mCD14) of 55 kDa. Both GPI-free sCD14 form (by proteolytic cleavage) and GPI-linked sCD14 form (by protease-mediated shedding) are found in the serum. The present inventors examined whether GPI-free or GPI-linked sCD14 was found in the urine. The analysis was performed by using Western blotting, and it can be confirmed that two forms of GPI-linked (56 kDa) and GPI-free (48 kDa) sCD14s were found in the urine obtained from RA and OA patients (FIG. 4(B)). In order to use antibodies bound to two forms of urinary sCD14s to examine whether an increase in urinary sCD14 levels occurs only in RA patients, or is a general phenomenon exhibited even in other forms of arthritis and autoimmune diseases, the concentrations of sCD14 obtained from patients with RA (n = 274), OA (n = 120), and SLE (n = 60) with no renal infiltration were measured by the ELISA method. The concentration of urinary sCD14 after correction for urine creatinine was higher in RA patients than in SLE and OA patients (FIG. 4(C)). In addition, the difference in urinary sCD14 levels was not exhibited with or without drugs (prednisolone, NSAIDs, DMARDs, and TNF-α inhibitors) in the RA patients (Table 3).

Diabetes mellitus (DM) and hypertension may affect the secretion of proteins into urine (urinary protein excretion), and thus an analysis was performed according to the expression of DM or hypertension. According to the results, RA patients had a higher level of urinary sCD14 than OA patients regardless of DM or hypertension (FIG. 4(D)). In particular, RA patients with DM exhibited the highest level of urinary sCD14 in the subgroup of RA patients, and RA patients with hypertension exhibited the second highest level (FIG. 4(D)).

**[Table 3]**

| | **Proteinuria (mg/day)** | | **P value** | **Urinary sCD14 (ng/ml)** | | **P value** |
|---|---|---|---|---|---|---|
| | **Users** | **Non-users** | | **Users** | **Non-users** | |
| Prednisolone | 92.8 (23.9-144.8) | 81.0 (21.8-162.4) | 0.795 | 179.9 (39.6-239.8) | 213.9 (49.8-313.0) | 0.322 |
| Nonsteroidal anti-inflammatory drugs (NSAIDs) | 94.4 (23.4-159.2) | 92.8 (25.2-98.1) | 0.790 | 188.4 (50.5-245.5) | 171.5 (40.2-218.7) | 0.611 |
| Methotrexate | 73.3 (23.5-133.1) | 118.7 (24.6-160.1) | 0.195 | 179.3 (39.4-235.6) | 198.4 (54.4-291.4) | 0.470 |
| Sulfasalazine | 86.4 (26.8-149.3) | 93.6 (23.2-211.5) | 0.850 | 198.7 (45.9-286.0) | 179.6 (39.2-238.9) | 0.501 |
| Hydroxychloroquine | 89.7 (25.7-150.1) | 96.2 (19.3-173.3) | 0.876 | 134.5 (44.8-278.7) | 199.0 (78.7-370.9) | 0.037 |
| Leflunomide | 98.3 (23.6-149.8) | 77.1 (22.3-159.6) | 0.556 | 194.4 (47.1-253.5) | 170.2 (38.2-228.3) | 0.342 |
| Azathioprine | 68.7 (19.9-108.9) | 95.0 (25.1-157.2) | 0.604 | 185.5 (87.8-283.8) | 186.1 (39.3-239.5) | 0.987 |
| Bucillamine | 77.4 (23.0-120.6) | 110.9 (24.7-171.8) | 0.351 | 187.7 (43.0-220.6) | 181.0 (47.8-235.8) | 0.802 |
| TNFα blocker | 81.5 (23.0-308.9) | 92.4 (24.3-145.5) | 0.864 | 196.8 (22.5-406.3) | 187.2 (43.7-239.7) | 0.858 |

### Example 4. Measurement of Correlation of Urinary sCD14 Concentration with RA Disease Activity Status

In order to evaluate the clinical importance of urinary sCD14 in RA, inflammatory indices of RA, including ESR, CRP, serum sCD14, and DAS28, were used to analyze whether the indices are associated with urinary sCD14 levels.

Disease activity status is defined by "Disease Activity Score 28-joint assessment (DAS28) score". The disease activity status is defined as low disease activity when the DAS28 score < 3.2, moderate disease activity when 3.2 ≤ DAS28 score < 5.1, and high disease activity when DAS28 score ≥ 5.1. The DAS28 score was calculated from the relationship equation including the ESR.

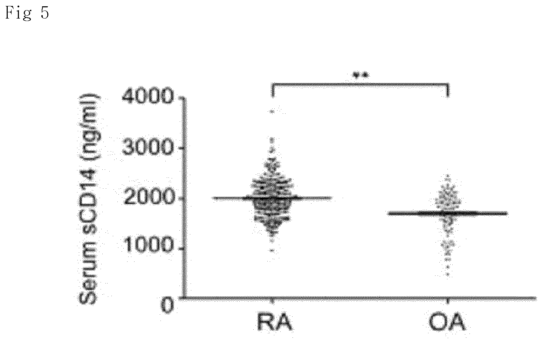

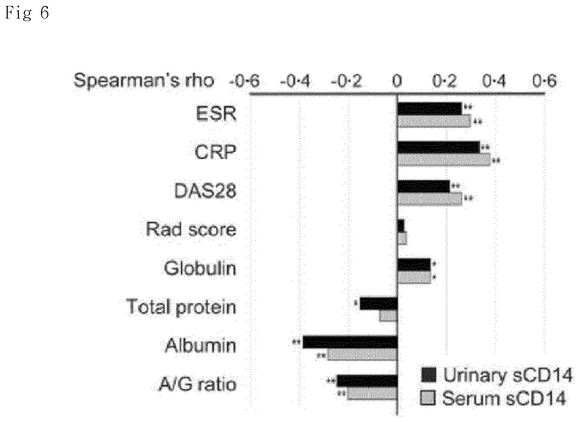

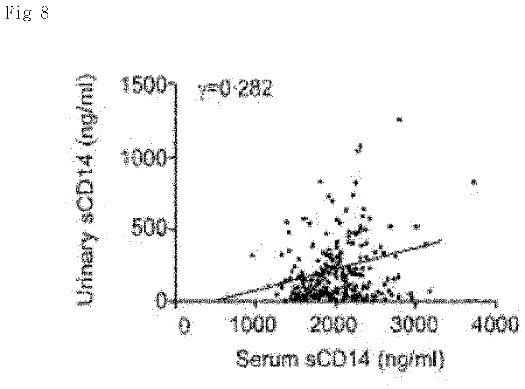

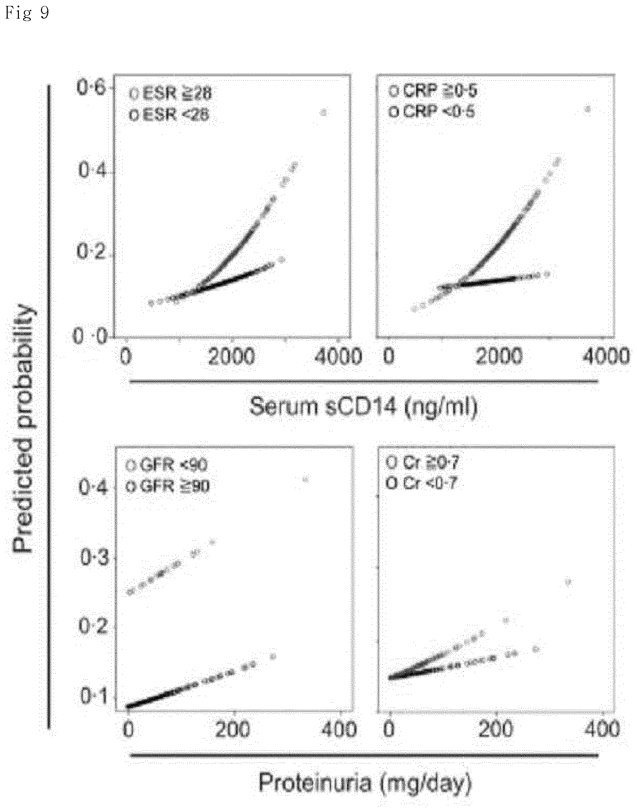

First, it was confirmed that the serum sCD14 concentration was higher in RA patients (n = 274) than OA patients (n = 120) (mean value [range]: 1991.0 [1732.1-2251.0] versus 1794.2 [1486.3-1999.1] ng/ml, P<0.001) (FIG. 5), and as a result of the analysis, urinary and serum sCD14 levels exhibited a strong positive correlation with ESR, CRP, and DAS28, and a negative correlation with serum albumin (FIG. 6). When RA patients were classified into three groups (high/moderate/low groups) according to the disease activity status according to DAS28, the urinary sCD14 level was increased in proportion to DAS28 (FIG. 7). Further, the urinary sCD14 exhibited a positive correlation with serum sCD14 (FIG. 8), and it could be seen from the result that the urinary sCD14 level may be used as a measure sensitive to the activity status of inflammatory response. According to the analysis stratified by ESR and CRP, serum sCD14 increased the possibility that the urinary sCD14 level would be elevated in the subgroup of RA patients with a high ESR (FIG. 9, upper panel). In addition, the probability that urinary sCD14 level would be elevated increased with rising serum sCD14 levels in patients with high ESR/CRP, compared to those with low ESR/CRP. These observations indicate that the increase in urinary sCD14 level results from progression from the systemic circulation to the renal system when the systemic inflammation is severe.

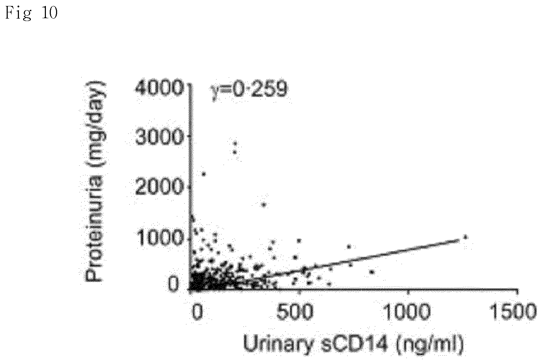

The present inventors confirmed that proteinuria occurred in RA patients, examined correlation with urinary sCD14 according to the degree of proteinuria, and observed that the urinary sCD14 level exhibits a strong positive correlation with the degree of proteinuria unlike the serum sCD14 (r = 0.259, P < 0.001) (FIG. 10). This means that urinary sCD14 is associated with the renal dysfunctions of RA patients. In this regard, the present inventors observed that RA patients with decreased renal functions had a higher probability that the urinary sCD14 levels would be elevated than RA patients with normal renal functions (FIG. 9, lower panel); OR=1.379 [1.059-1.944] per one SD increase in proteinuria for cases with GFR <90 ml/min/1.732, P=0.031; OR=1.417 [1.083-1.853] per one SD increase in proteinuria for cases with serum creatinine (Cr)³ 0.7 mg/dl, P=0.010.

### Example 5. Urinary sCD14 as Marker which Predicts RA Activity

In order to evaluate the diagnostic power of urinary sCD14 in RA patients with high RA activity (DAS28 > 5.1), the ROC analysis was performed (FIG. 11). In this analysis, CRP was used as a parameter useful for predicting the disease activity of RA, which is deeply associated with DAS28. The serum CRP exhibited an area under the plasma concentration-time curve (AUC, a measure of diagnostic power) of 0.74 [0.67-0.81, P < 0.001], the urinary sCD14 exhibited 0.71 [0.63-0.79, P < 0.001] which was a level similar to the AUC, and it could be seen that the urinary sCD14 is at a level similar to CRP in predicting high RA activities (DAS28 > 5.1). Furthermore, the ROC analysis exhibited that the urinary sCD14 had higher sensitivity in a region exhibiting a specificity of 80% or more than CRP, meaning that the urinary sCD14 could forecast the disease activity status more complementarily than when measured with CRP.

Based on these results, the present inventors used the ratio of serum CRP to urinary sCD14 as a new complex evaluation reference of CRP and sCD14, and also determined a cutoff value (0.06) of the evaluation reference to minimize the sum of false positivity and negative errors in the ROC. When the measurement is compositely performed, (when the cut-off was set to 0.06) the sensitivity, specificity, positive predictive value (PPV), and negative predictive value (NPV), in which an RA patient is determined to have a high disease activity status, correspond to 77.2%, 68.9%, 44.4%, and 90.4%, respectively. It could be seen that these values are larger than those when CRP or urinary sCD14 is used alone (Table 4).

**[Table 4]**

| | CRP(95% CI) | sCD14(95% CI) | CRP/sCD14 (95% CI) |
|---|---|---|---|
| Sensitivity | 70.8% (64.9-76.1) | 68.4% (62.0-74.3) | 77.2% (71.2-82.3) |
| Specificity | 66.8% (60.8-72.4) | 62.7% (56.1-68.9) | 68.9% (62.5-74.7) |
| PPV | 40.4% (34.5-46.5) | 37.1% (31.0-43.7) | 44.4% (38.0-51.1) |
| NPV | 87.8% (83.2-91.4) | 86.0% (80.8-90.1) | 90.4% (85.7-93.8) |
| Cut-off | 6ng/ml | 102ng/ml | 0.06 |

When these results are synthesized, it can be seen that the urinary sCD14 itself is a non-invasive biomarker, has a diagnostic power which is at a level equivalent to that of serum CRP, and may be used as a biomarker with better activity when combined with serum CRP.

### Example 6. Study of Correlations of Urinary AGP1, AGP2, and sCD14 Concentrations with RA Disease Activity Status

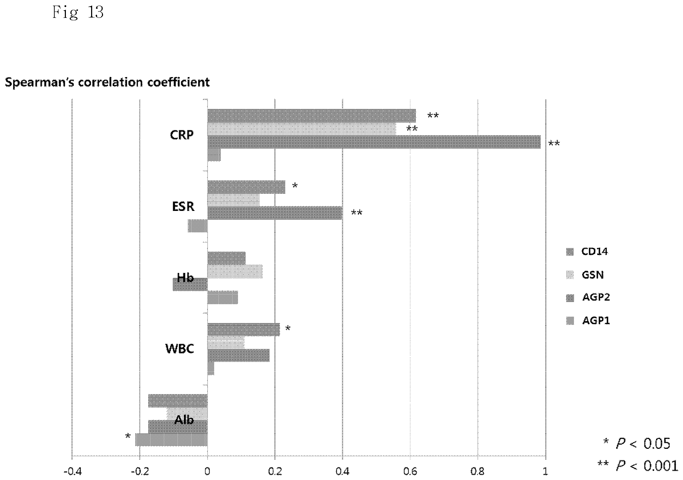

In order to evaluate the possibility that urinary AGP1, AGP2, and sCD14 in RA may be used as an inflammatory marker, which may reflect the disease activity status, the correlations of these proteins with ESR, CRP, DAS28, hemoglobin, number of white blood cells (WBCs), and albumin were analyzed. RA patients (FIG. 12) and control patients (FIG. 13) were each divided to perform a correlation analysis, and both AGP2 and sCD14 in the two groups exhibited a significant correlation with the inflammatory markers. In particular, AGP2 exhibited a very strong correlation with serum CRP (p = 0.664 and P < 0.001 in the total number of patients, p = 0.602 and P < 0.001 in RA, and p = 0.985 and P < 0.001 in the control patients).

Some of the RA patients may exhibit a high disease activity status even in low CRP concentration in some cases, and thus, there is a need for a new inflammatory disease activity status marker which may represent a disease activity status (DAS28) from these patients. In the present invention, as a result of performing an analysis stratified by dividing the RA patients into a group of patients having a normal CRP concentration and a group of patients having no normal CRP concentration, sCD14 exhibited a good correlation with DAS28 in the normal CRP group (Table 5). Meanwhile, AGP2 exhibited a strong correlation with CRP in both the normal CRP group and the elevated CRP group (Table 6). The aforementioned results exhibit that sCD14 may reflex DAS28 from the complementary viewpoint of serum CRP. Further, AGP2 is a very good reflection factor of serum CRP, indicating that the disease activity status may be evaluated only by a urine test without any collection of a blood sample.

**[Table 5]**

| | ESR | CRP | DAS28 | WBC | Hemoglobin | Albumin |
|---|---|---|---|---|---|---|
| Unadjusted | | | | | | |
| AGP1 | -0.093 | -0.039 | 0.001 | -0.004 | 0.043 | -0.016 |
| AGP2 | 0.077 | 0.480 | 0.022 | 0.110 | -0.093 | -0.244 |
| GSN | 0.098 | 0.136 | -0.100 | 0.070 | 0.113 | -0.184 |
| CD14 | 0.351 | 0.048 | 0.390 | 0.150 | 0.007 | -0.424 |

| Ucr-adjusted | | | | | | |
|---|---|---|---|---|---|---|
| AGP1 | -0.019 | 0.022 | -0.019 | -0.045 | -0.122 | -0.235 |
| AGP2 | 0.142 | 0.094 | 0.047 | 0.159 | -0.244 | -0.139 |
| GSN | 0.207 | 0.136 | -0.025 | 0.189 | -0.000 | -0.189 |
| CD14 | 0.368 | 0.005 | 0.267 | 0.244 | -0.214 | -0.309 |
| Spearman's correlation coefficient was used to determine p value | | | | | | |

**[Table 6]**

| | ESR | CRP | DAS28 | WBC | Hemoglobin | Albumin |
|---|---|---|---|---|---|---|
| Unadjusted | | | | | | |
| AGP1 | -0.093 | -0.039 | 0.001 | -0.004 | 0.043 | -0.016 |
| AGP2 | 0.230 | 0.613 | 0.118 | 0.123 | -0.229 | -0.405 |
| GSN | -0.129 | -0.036 | -0.121 | -0.125 | 0.045 | -0.106 |
| CD14 | 0.158 | 0.344 | 0.134 | 0.030 | -0.065 | -0.384 |

| Ucr-adjusted | | | | | | |
|---|---|---|---|---|---|---|
| AGP1 | -0.006 | 0.024 | 0.155 | 0.092 | 0.035 | -0.102 |
| AGP2 | 0.015 | 0.244 | -0.015 | -0.076 | -0.021 | -0.377 |
| GSN | -0.142 | -0.021 | -0.081 | -0.110 | 0.068 | -0.240 |
| CD14 | 0.157 | 0.155 | 0.117 | 0.118 | -0.094 | -0.254 |
| Spearman's correlation coefficient was used to determine p value | | | | | | |

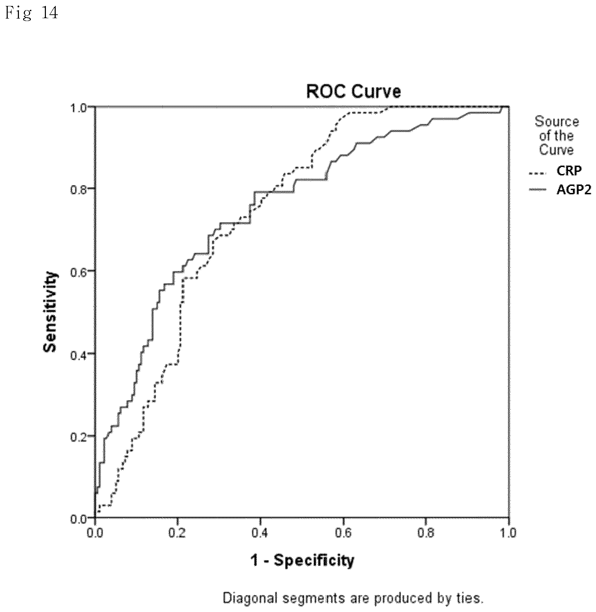

In order to approve that urinary AGP2 has power as a marker which reflects the disease activity status in RA patients, a group with DAS28 ≥ 5.1 was defined as a group with high disease activity status, and the ROC analysis was performed (FIG. 14). In the analysis, AGP2 exhibited AUC = 0.645 [0.685-0.822, P < 0.001], which is a result close to a serum CRP AUC = 0.659 [0.680-0.803, P < 0.001] (Table 7). When the cut-off value of AGP2 was set to 0.3555 ng/ml, a sensitivity of 71.6% and a specificity of 70.0%, which may differentiate a group of patients with high disease activity status, exhibited possibility as very good indices (Table 8).

**[Table 7]**

| | AUC | P | 95% CI |
|---|---|---|---|
| CRP | 0.659 | <0.001 | 0.680-0.803 |
| AGP2 | 0.645 | <0.001 | 0.685-0.822 |

**[Table 8]**

| Cut-Off | Sensitivity | Specificity |
|---|---|---|
| 0.3555 | 71.6 | 70.0 |

### Example 7. Urinary AGP1, AGP2, and sCD14 as Markers which Predict RA Diagnosis

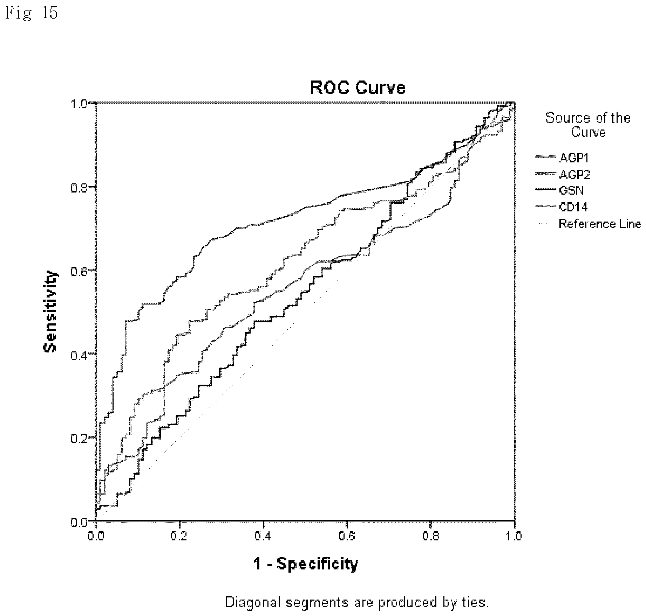

In order to see the possibility of RA differential diagnosis in patients with arthritis, the diagnostic power-ROC analysis of urinary sCD14, AGP1, AGP2, and GSN was performed in the patients (FIG. 15). Since it is a goal to develop a diagnostic tool which can diagnose RA by using only urine in the future, the analysis was performed on the assumption that there is no information associated with renal functions (that is, without any adjustment for renal correction). In the analysis, AGP2 was 0.713 [0.659-0.767, P < 0.001], exhibiting the best AUC value, and sCD14 was 0.616 [0.555-0.678, P < 0.001] (Table 9).

AGP2 had disease diagnostic sensitivity of 61.0% and specificity of 76.1% when the cut-off value was set to 0.3285 ng/ml, and sCD14 has a sensitivity of 55.2% and a specificity of 66.3% when the cut-off was set to 116.944 ng/ml (Table 10). These observations suggested the possibility that urine is very promising as a diagnostic screening tool of RA in terms of levelbecause these proteins exhibit specificity at a relatively high level while exhibiting sensitivity better than the diagnostic power (a sensitivity of 48 to 63% and a specificity of 88 to 96%) of a rheumatoid factor(RF) or an anti-cyclic citrullinated antibody (anti-CCP antibody), which is a diagnostic associated antibody known in the related art.

**[Table 9]**

| | AUC | P | 95% CI |
|---|---|---|---|
| AGP1 | 0.550 | 0.102 | 0.493-0.620 |
| AGP2 | 0.713 | <0.001 | 0.659-0.767 |
| GSN | 0.541 | 0.235 | 0.474-0.608 |
| CD14 | 0.616 | 0.001 | 0.555-0.678 |

**[Table 10]**

| | Cut-Off | Sensitivity | Specificity |
|---|---|---|---|
| AGP1(ng/ml) | 0.3670 | 45.4 | 74.1 |
| AGP2(ng/ml) | 0.3285 | 61.0 | 76.1 |
| GSN(ng/ml) | 1.4881 | 44.3 | 66.4 |
| CD14(ng/ml) | 116.9440 | 55.2 | 66.3 |

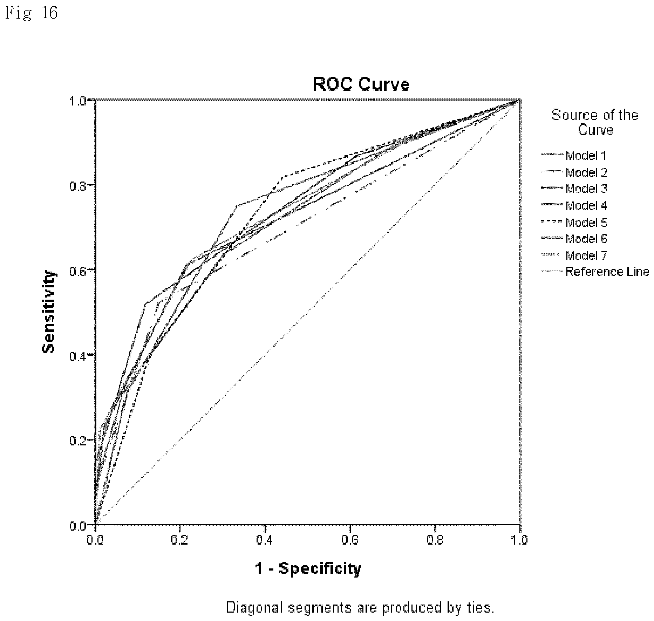

Further, since urinary AGP1, AGP2, and sCD14 exhibit a mutual correlation in the ROC analysis, it is judged that a combination of these proteomes may suggest a higher diagnostic yield, and thus, various combinations of the diagnostic power-ROC analyses were performed (FIG. 16).

**[Table 11]**

| | Combination The number in the parentheses indicates a cut-off value (ng/ml) |
|---|---|
| Model 1 | AGP1(0.367) + AGP2(0.231) + GSN(15.3288) + CD14(139.95) |
| Model 2 | AGP1(0.360) + AGP2(0.20074) + CD14(116.944) |
| Model 3 | AGP1(0.4066) + AGP2(0.3528) + CD14(228.2) |
| Model 4 | AGP1(0.4328) + AGP2(0.6612) + CD14(621.25) |
| Model 5 | AGP2(0.20074) + CD14(116.944) |
| Model 6 | AGP2(0.231) + CD14(139.95) |
| Model 7 | AGP1(0.3175) + AGP2(0.337) |

In the various combination models, Model 3, in which three urinary proteins, that is, AGP1, AGP2, and CD14 were combined, had an AUC of 0.727 [0.671-0.783, P < 0.001], which is the best result (Table 12). Model 3 was composed of a total sum of points obtained when the cut-off of each protein was set to 0.4066 ng/ml, 0.3528 ng/ml, and 228.2 ng/ml for AGP1, AGP2, and sCD, respectively, and then a point of 1 is given for a value which is the cut-off or more and a point of 0 is given for a value which is less than the cut-off.

In addition, Model 5 [composed of AGP2 (cut-off = 0.20074 ng/ml) and sCD14 (cut-off = 116.944 ng/ml)] and Model 6 [composed of AGP2 (cut-off = 0.231 ng/ml) and sCD14 (cut-off = 139.95 ng/ml)], which were composed of only two proteinsin consideration of economic effects against the costs also exhibited good diagnostic power as an AUC of 0.712 [0.650-0.774, P < 0.001] and 0.718 [0.657-0.778, P < 0.001], respectively (Table 12). When both AGP2 and sCD14 were positive based on the cut-off of Model 6, the sensitivity and specificity were 84.8% and 61.1%, respectively in the diagnosis of RA. In addition, when both AGP1 and AGP2 were positive in the combination thereof, the sensitivity and specificity were 91.7% and 70.0%, respectively in the diagnosis of RA (Table 13).

**[Table 12]**

| | AUC | P | 95% CI |
|---|---|---|---|
| Model 1 | 0.698 | <0.001 | 0.638-0.757 |
| Model 2 | 0.721 | <0.001 | 0.664-0.778 |
| Model 3 | 0.727 | <0.001 | 0.671-0.783 |
| Model 4 | 0.699 | <0.001 | 0.641-0.757 |
| Model 5 | 0.712 | <0.001 | 0.650-0.774 |
| Model 6 | 0.718 | <0.001 | 0.657-0.778 |
| Model 7 | 0.693 | <0.001 | 0.635-0.751 |

**[Table 13]**

| | Cut-Off | Sensitivity | Specificity |
|---|---|---|---|
| AGP2(ng/ml) CD14(ng/ml) 2 positive | 0.213 | 84.8 | 61.1 |
| | 139.95 | | |
| AGP1(ng/ml) AGP2(ng/ml) 2 positive | 0.3175 | 91.7 | 70.0 |
| | 0.337 | | |

When the aforementioned results are synthesized, it can be seen that AGP1, AGP2, and sCD14 are non-invasive biomarkers and have diagnostic power at a level equivalent to a serum autoantibody (RF or ACPA), and the mutual combination of these proteins may be used as a better biomarker for diagnosis.
## Claims

1. Use of measuring the concentrations of soluble CD14 (sCD14) and alpha 1 acid glycoprotein 2 (AGP2) in combination in a urine sample for the diagnosis and activity evaluation of rheumatoid arthritis.

2. The use of claim 1, further comprising measuring the concentration of alpha-1-acid glycoprotein 1 (AGP1).

3. The use of claim 1 or 2, wherein the agent for measuring the concentrations of the proteins is an antibody specific to one or more selected from the group consisting of sCD14, AGP1, and AGP2.

4. The use of claim 3, wherein the antibody is a monoclonal antibody or a polyclonal antibody.

5. The use of claim 1 or 2, further comprising:
measuring the concentrations of one or more proteins selected from a group consisting of immunoglobulin heavy constant mu (IGHM), gelsolin (GSN), serpin peptidase inhibitor, clade A (alpha-1 antiproteinase, antitrypsin), member 3 (SERPINA3), alpha-2-glycoprotein 1, zinc (AZGP1), coactosin-like F-actin binding protein 1 (COTL1), haptoglobin isoform 2 preproprotein (HPR), serpin peptidase inhibitor, clade A (alpha-1 antiproteinase, antitrypsin), member 7 (SERPINA7), cathepsin A (CTSA), and glucosamine(N-acetyl)-6-sulfatase (GNS).

6. A kit for diagnosis or activity evaluation of rheumatoid arthritis, comprising:
agents to measure the concentration of sCD14 and AGP2 in combination, and optionally AGP1 in combination.

7. A method for providing information for diagnosing or evaluating activity of rheumatoid arthritis, the method comprising:
measuring the concentrations of proteins sCD14 and AGP2 from a urine sample in combination.

8. The method of claim 7, further comprising measuring the concentration of AGP1.

9. The method of claim 7 or 8, wherein the measuring of the concentrations of the proteins is performed by using an antibody specific to sCD14, AGP1 or AGP2.

10. The method of claim 9, wherein the antibody is a monoclonal antibody or a polyclonal antibody.

11. The method of claim 7, wherein the measuring of the concentrations of the proteins is performed by using Western blot, enzyme linked immunosorbent assay (ELISA), immunoprecipitation assay, complement fixation assay, fluorescence activated cell sorter (FACS), or protein chip.

12. The method of claim 7 or 8, wherein one or more proteins selected from the group consisting of GSN, SERPINA3, AZGP1, COTL1, HPR, SERPINA7, CTSA, and GNS are further detected.

## Patentansprüche

1. Verwendung des Messens der Konzentrationen von löslichem CD14 (sCD14) und Alpha-1-Säure-Glykoprotein 2 (AGP2) in Kombination in einer Urinprobe zur Diagnose und Aktivitätsbewertung von rheumatoider Arthritis.

2. Verwendung nach Anspruch 1, weiterhin umfassend das Messen der Konzentration von Alpha-1-Säure-Glykoprotein 1 (AGP1).

3. Verwendung nach Anspruch 1 oder 2, wobei der Wirkstoff zum Messen der Konzentrationen der Proteine ein Antikörper ist, der für einen oder mehrere, ausgewählt aus der Gruppe, bestehend aus sCD14, AGP1 und AGP2, spezifisch ist.

4. Verwendung nach Anspruch 3, wobei der Antikörper ein monoklonaler Antikörper oder ein polyklonaler Antikörper ist.

5. Verwendung nach Anspruch 1 oder 2, weiterhin umfassend:
das Messen der Konzentrationen von einem oder mehreren Proteinen, ausgewählt aus einer Gruppe, bestehend aus IGHM (Immunglobulin *heavy constant mu*), Gelsolin (GSN), Serpin-Peptidase-Inhibitor, Gruppe A (Alpha-1 Antiproteinase, Antitrypsin), Mitglied 3 (SERPINA3), Alpha-2-Glycoprotein 1, Zink (AZGP1), Koaktosinähnlichem, F-Actin bindendem Protein 1 (COTL1), Haptoglobin-Isoform 2-Präprotein (HPR), Serpin-Peptidase-Inhibitor, Gruppe A (Alpha-1 Antiproteinase, Antitrypsin), Mitglied 7 (SERPINA7), Cathepsin A (CTSA) und Glucosamin(N-Acetyl)-6-Sulfatase (GNS).

6. Kit zur Diagnose oder Aktivitätsbewertung von rheumatoider Arthritis, umfassend:
Wirkstoffe zur Messung der Konzentration von sCD14 und AGP2 in Kombination und wahlweise AGP1 in Kombination.

7. Verfahren zur Bereitstellung von Informationen zur Diagnose oder Aktivitätsbewertung von rheumatoider Arthritis, wobei das Verfahren Folgendes umfasst:
das Messen der Konzentrationen der Proteine sCD14 und AGP2 aus einer Urinprobe in Kombination.

8. Verfahren nach Anspruch 7, weiterhin umfassend das Messen der Konzentration von AGP1.

9. Verfahren nach Anspruch 7 oder 8, wobei das Messen der Konzentrationen der Proteine durch Verwenden eines Antikörpers durchgeführt wird, der spezifisch für sCD14, AGP1 oder AGP2 ist.

10. Verfahren nach Anspruch 9, wobei der Antikörper ein monoklonaler Antikörper oder ein polyklonaler Antikörper ist.

11. Verfahren nach Anspruch 7, wobei das Messen der Konzentrationen der Proteine durch Verwendung von Western Blot, enzymgekoppeltem Immunadsorptionsassay (ELISA), Immunpräzipitationsassay, Komplementbindungsassay, Fluoreszenz-aktivierten Zellsortierer (FACS) oder Protein-Chip durchgeführt wird.

12. Verfahren nach Anspruch 7 oder 8, wobei eines oder mehrere Proteine, ausgewählt aus der Gruppe, bestehend aus GSN, SERPINA3, AZGP1, COTL1, HPR, SERPINA7, CTSA und GNS, weiterhin detektiert werden.

## Revendications

1. Utilisation de la mesure des concentrations de CD14 soluble (sCD14) et de l'alpha-1 acide glycoprotéine 2 (AGP2) en combinaison dans un échantillon d'urine pour le diagnostic et l'évaluation de l'activité de la polyarthrite rhumatoïde.

2. Utilisation selon la revendication 1, comportant en outre la mesure de la concentration de l'alpha-1 acide glycoprotéine 1 (AGP1).

3. Utilisation selon la revendication 1 ou 2, l'agent pour mesurer les concentrations des protéines étant un anticorps spécifique à un ou plusieurs choisi parmi le groupe constitué de sCD14, AGP1 et AGP2.

4. Utilisation selon la revendication 3, l'anticorps étant un anticorps monoclonal ou un anticorps polyclonal.

5. Utilisation selon la revendication 1 ou 2, comportant en outre:
la mesure des concentrations d'une ou de plusieurs protéines choisie parmi un groupe constituée de l'IGHM (immunoglobuline *heavy constant mu*), de gelsoline (GSN), de l'inhibiteur de la peptidase de serpine, du clade A (alpha-1 antiprotéinase, antitrypsine), du membre 3 (SERPINA3), de l'alpha-2 glycoprotéine 1, de zinc (AZGP1), de la protéine 1 de liaison à F-actine similaire à la coactosine (COTL1), de la préprotéine de haptoglobine isoforme 2 (HPR), de l'inhibiteur de la peptidase de serpine, du clade A (alpha-1 antiprotéinase, antitrypsine), du membre 7 (SERPINA7), de la cathepsine A (CTSA) et de la glucosamine(N-acétyle)-6-sulfatase (GNS).

6. Kit pour le diagnostic ou l'évaluation de l'activité de la polyarthrite rhumatoïde, comportant:
des agents pour mesurer la concentration de sCD14 et de AGP2 en combinaison, et facultativement de AGP1 en combinaison.

7. Procédé pour fournir des informations pour le diagnostic ou l'évaluation de l'activité de la polyarthrite rhumatoïde, le procédé comportant:
la mesure des concentrations des protéines sCD14 et AGP2 à partir d'un échantillon d'urine en combinaison.

8. Procédé selon la revendication 7, comportant en outre la mesure de la concentration de AGP1.

9. Procédé selon la revendication 7 ou 8, la mesure des concentrations des protéines étant effectuée en utilisant un anticorps spécifique à sCD14, AGP1 ou AGP2.

10. Procédé selon la revendication 9, l'anticorps étant un anticorps monoclonal ou un anticorps polyconal.

11. Procédé selon la revendication 7, la mesure des concentrations des protéines étant effectuée en utilisant le Western blot, l'essai immuno-enzymatique (ELISA), l'essai d'immunoprécipitation, l'essai de fixation du complément, le triage de cellules activé par fluorescence (FACS), ou une puce protéique.

12. Procédé selon la revendication 7 ou 8, une ou plusieurs protéines choisie parmi le groupe constitué de GSN, SERPINA3, AZGP1, COTL1, HPR, SERPINA7, CTSA et GNS étant détectée en outre.


<110> CATHOLIC UNIVERSITY INDUSTRY-ACADEMIC COOPERATION FOUNDATION
<120> Biomarkers for diagnosing rheumatoid arthritis or assessing
   rheumatoid arthritis disease activity
<130> OPP20142285KR
<150> KR10-2013-0071739
   <151> 2013-06-21
<160> 3
<170> Kopatent In 1.71
<210> 1
   <211> 375
   <212> PRT
   <213> Homo sapiens
<220>
   <221> PEPTIDE
   <222> (1)..(375)
   <223> sCD14
<400> 1
   Met Glu Arg Ala Ser Cys Leu Leu Leu Leu Leu Leu Pro Leu Val His
   1 5 10 15
   Val Ser Ala Thr Thr Pro Glu Pro Cys Glu Leu Asp Asp Glu Asp Phe
   20 25 30
   Arg Cys Val Cys Asn Phe Ser Glu Pro Gln Pro Asp Trp Ser Glu Ala
   35 40 45
   Phe Gln Cys Val Ser Ala Val Glu Val Glu Ile His Ala Gly Gly Leu
   50 55 60
   Asn Leu Glu Pro Phe Leu Lys Arg Val Asp Ala Asp Ala Asp Pro Arg
   65 70 75 80
   Gln Tyr Ala Asp Thr Val Lys Ala Leu Arg Val Arg Arg Leu Thr Val
   85 90 95
   Gly Ala Ala Gln Val Pro Ala Gln Leu Leu Val Gly Ala Leu Arg Val
   100 105 110
   Leu Ala Tyr Ser Arg Leu Lys Glu Leu Thr Leu Glu Asp Leu Lys Ile
   115 120 125
   Thr Gly Thr Met Pro Pro Leu Pro Leu Glu Ala Thr Gly Leu Ala Leu
   130 135 140
   Ser Ser Leu Arg Leu Arg Asn Val Ser Trp Ala Thr Gly Arg Ser Trp
   145 150 155 160
   Leu Ala Glu Leu Gln Gln Trp Leu Lys Pro Gly Leu Lys Val Leu Ser
   165 170 175
   Ile Ala Gln Ala His Ser Pro Ala Phe Ser Cys Glu Gln Val Arg Ala
   180 185 190
   Phe Pro Ala Leu Thr Ser Leu Asp Leu Ser Asp Asn Pro Gly Leu Gly
   195 200 205
   Glu Arg Gly Leu Met Ala Ala Leu Cys Pro His Lys Phe Pro Ala Ile
   210 215 220
   Gln Asn Leu Ala Leu Arg Asn Thr Gly Met Glu Thr Pro Thr Gly Val
   225 230 235 240
   Cys Ala Ala Leu Ala Ala Ala Gly Val Gln Pro His Ser Leu Asp Leu
   245 250 255
   Ser His Asn Ser Leu Arg Ala Thr Val Asn Pro Ser Ala Pro Arg Cys
   260 265 270
   Met Trp Ser Ser Ala Leu Asn Ser Leu Asn Leu Ser Phe Ala Gly Leu
   275 280 285
   Glu Gln Val Pro Lys Gly Leu Pro Ala Lys Leu Arg Val Leu Asp Leu
   290 295 300
   Ser Cys Asn Arg Leu Asn Arg Ala Pro Gln Pro Asp Glu Leu Pro Glu
   305 310 315 320
   Val Asp Asn Leu Thr Leu Asp Gly Asn Pro Phe Leu Val Pro Gly Thr
   325 330 335
   Ala Leu Pro His Glu Gly Ser Met Asn Ser Gly Val Val Pro Ala Cys
   340 345 350
   Ala Arg Ser Thr Leu Ser Val Gly Val Ser Gly Thr Leu Val Leu Leu
   355 360 365
   Gln Gly Ala Arg Gly Phe Ala
   370 375
<210> 2
   <211> 201
   <212> PRT
   <213> Homo sapiens
<220>
   <221> PEPTIDE
   <222> (1)..(201)
   <223> AGP1
<400> 2
   Met Ala Leu Ser Trp Val Leu Thr Val Leu Ser Leu Leu Pro Leu Leu
   1 5 10 15
   Glu Ala Gln Ile Pro Leu Cys Ala Asn Leu Val Pro Val Pro Ile Thr
   20 25 30
   Asn Ala Thr Leu Asp Arg Ile Thr Gly Lys Trp Phe Tyr Ile Ala Ser
   35 40 45
   Ala Phe Arg Asn Glu Glu Tyr Asn Lys Ser Val Gln Glu Ile Gln Ala
   50 55 60
   Thr Phe Phe Tyr Phe Thr Pro Asn Lys Thr Glu Asp Thr Ile Phe Leu
   65 70 75 80
   Arg Glu Tyr Gln Thr Arg Gln Asp Gln Cys Ile Tyr Asn Thr Thr Tyr
   85 90 95
   Leu Asn Val Gln Arg Glu Asn Gly Thr Ile Ser Arg Tyr Val Gly Gly
   100 105 110
   Gln Glu His Phe Ala His Leu Leu Ile Leu Arg Asp Thr Lys Thr Tyr
   115 120 125
   Met Leu Ala Phe Asp Val Asn Asp Glu Lys Asn Trp Gly Leu Ser Val
   130 135 140
   Tyr Ala Asp Lys Pro Glu Thr Thr Lys Glu Gln Leu Gly Glu Phe Tyr
   145 150 155 160
   Glu Ala Leu Asp Cys Leu Arg Ile Pro Lys Ser Asp Val Val Tyr Thr
   165 170 175
   Asp Trp Lys Lys Asp Lys Cys Glu Pro Leu Glu Lys Gln His Glu Lys
   180 185 190
   Glu Arg Lys Gln Glu Glu Gly Glu Ser
   195 200
<210> 3
   <211> 201
   <212> PRT
   <213> Homo sapiens
<220>
   <221> PEPTIDE
   <222> (1)..(201)
   <223> AGP2
<400> 3
   Met Ala Leu Ser Trp Val Leu Thr Val Leu Ser Leu Leu Pro Leu Leu
   1 5 10 15
   Glu Ala Gln Ile Pro Leu Cys Ala Asn Leu Val Pro Val Pro Ile Thr
   20 25 30
   Asn Ala Thr Leu Asp Arg Ile Thr Gly Lys Trp Phe Tyr Ile Ala Ser
   35 40 45
   Ala Phe Arg Asn Glu Glu Tyr Asn Lys Ser Val Gln Glu Ile Gln Ala
   50 55 60
   Thr Phe Phe Tyr Phe Thr Pro Asn Lys Thr Glu Asp Thr Ile Phe Leu
   65 70 75 80
   Arg Glu Tyr Gln Thr Arg Gln Asn Gln Cys Phe Tyr Asn Ser Ser Tyr
   85 90 95
   Leu Asn Val Gln Arg Glu Asn Gly Thr Val Ser Arg Tyr Glu Gly Gly
   100 105 110
   Arg Glu His Val Ala His Leu Leu Phe Leu Arg Asp Thr Lys Thr Leu
   115 120 125
   Met Phe Gly Ser Tyr Leu Asp Asp Glu Lys Asn Trp Gly Leu Ser Phe
   130 135 140
   Tyr Ala Asp Lys Pro Glu Thr Thr Lys Glu Gln Leu Gly Glu Phe Tyr
   145 150 155 160
   Glu Ala Leu Asp Cys Leu Cys Ile Pro Arg Ser Asp Val Met Tyr Thr
   165 170 175
   Asp Trp Lys Lys Asp Lys Cys Glu Pro Leu Glu Lys Gln His Glu Lys
   180 185 190
   Glu Arg Lys Gln Glu Glu Gly Glu Ser
   195 200